# EUROPEAN PATENT SPECIFICATION

(11) **EP 0 552 280 B1**
(45) Date of publication and mention of the grant of the patent: **28.06.1995**
(21) Application number: 91919581.8
(22) Date of filing: 09.10.1991
(51) Int. Cl.: C12N 15/63, C12N 15/11

(54) **INHIBITION OF VIRAL REPLICATION**
HEMMUNG DER VIRALEN REPLIKATION
INHIBITION DE REPLICATION VIRALE

(30) Priority: 09.10.1990 US 595253
(43) Date of publication of application: 28.07.1993
(73) Proprietor: NeXstar Pharmaceuticals, Inc., Boulder, CO 80301 (US)
(72) Inventor: SCOLARO, Michael, J., Beverly Hills, CA 90210 (US); SULLIVAN, Sean, M., Shaker Heights, OH 44120 (US)
(74) Representative: Brown, David Leslie
(86) International application number: US9107550
(87) International publication number: WO9206192

(56) References cited:
- WO-A-88/10311
- WO-A-89/08146
- DE-A- 3 742 049
- Journal of Virology, volume 62, no. 10, October 1988, American Society for Microbiology (Baltimore, US) J.A. Zaia et al.: "Inhibition of human immunodeficiency virus by using an oligonucleoside methylphosphonate targeted to the tat-3 gene", pp. 3914-3917, s. the abstract; p. 3916
- Biochemical and Biophysical Research Communications, volume 169, no. 2, 15 June 1990, Academic Press. Inc. (Duluth, Minnesota, US) G. Sczakiel et al.:"Specific inhibition of human immunodeficiency virus type 1 replication by RNA transcribed in sense and antisense orientation from the 5'-leader/gag region",pages 643-651, see the whole article
- Proceedings of the National Academy of Sciences of USA, volume 86, no. 20, October 1989 (Washington, DC, US) S. Agrawal et al.: "Inhibition of humanimmuno deficiency virus in early infected and chronically infected cells byantisense oligodeoxynucleotides and their phosphorothioate analogues", pages7790-7794, see page 7791, table 1; oligonucleotides 1 and 3

## Description

### Field of the Invention

This invention relates to the fields of biochemistry and medicine, more particularly to the delivery to target cells of oligonucleotides complementary to viral genes for the purpose of inhibiting viral replication.

### Background of the Invention

A virus is an infectious agent composed of a protein sheath surrounding a nucleic acid core, which is capable of infecting a plant, animal or bacteria. Viruses are characterized by having a total dependence on a living host cell for reproduction, and by a lack of independent metabolism. After passing into the host cell, during an early stage of viral replication, the virion (*i.e*., the complete, mature virus particle) disintegrates, freeing elements that direct the host cell's metabolism in the process of reproducing the virus. Specifically, the nucleic acid core (RNA or DNA) is released as the basic infectious material of the virus. Generally, virions containing DNA are referred to by the common term viruses, or more properly DNA viruses. Those containing RNA in the virion core, and whose genomic RNA is copied to form DNA which is integrated into the host DNA, are referred to as retroviruses or RNA viruses.

When DNA from a DNA virus is released in the host cell, this DNA is integrated into the cellular DNA for later reproduction of the virion which, often after destroying the host cell is expelled to continue the infection in other cells. If positive strand RNA is the genetic material of the virus, this RNA is copied to DNA by one of the viral proteins (reverse transcriptase) and the resulting viral DNA is integrated and reproduced by the reproductive mechanism of the host cell as described in the preceding sentence. For RNA viruses in which the genomic material does not integrate into the host DNA, both positive and negative stranded viral RNA are ultimately translated into structural proteins and copied into host cell genomic RNA by a viral RNA-dependent RNA polymerase for packaging into virions with no requirement for integration into cellular DNA.

A virus which is of much concern at present is the causative agent of autoimmune deficiency syndrome, which has been denominated as the human immunodeficiency virus (HIV). This is an RNA virus whose genome is copied and becomes integrated into the host DNA. This virus has been shown to infect cells of the immune system, predominantly T cells, monocytes and neural tissue, resulting in a significant attenuation or destruction of immune response in the human body.

HIV first infects cells of the immune system by binding to the CD4 protein expressed on the membrane surface of the cell. The binding event initiates fusion of the viral membrane with the plasma membrane of the cell, releasing the viral core into the cytoplasm. The viral core contains a protein (reverse transcriptase) which copies the viral RNA into viral DNA. The viral DNA complexed with other viral proteins migrates to the cellular nucleus where the viral DNA is integrated into the genomic DNA of the host cell. This is termed the latent state of the infection because no virus particles are produced.

In the acute state of the infection, viral RNA is produced by transcription of the integrated viral DNA by a cellular enzyme termed RNA polymerase II. This RNA is processed into genomic RNA and messenger RNA. The messenger RNA is translated into structural proteins necessary for infectious virion assembly. The virions, composed of genomic RNA and the viral structural proteins, are assembled and released from the cell by budding. In addition, the HIV viral genome codes for six accessory proteins which are not comprised within the virion but which are necessary for infectious virion assembly. These proteins are referred to as *tat* (*trans*-activating protein), *rev* (a regulator of virion protein expression), *nef*, *vif*, *vpr*, and *vpu*. The nucleic acid coding sequences for these proteins are encoded within the gene sequence coding for the envelope protein, which is a structural (as opposed to regulatory) virion protein. The envelope gene DNA is transcribed by RNA polymerase II to RNA, which is excised and ligated, leading to the production of mature messenger RNA which is transported to the cytoplasm and translated into the accessory proteins. The *tat* protein is a *trans*-activation protein which amplifies the production of viral RNA by binding to the promoter region of the integrated viral DNA. Furthermore, this protein also increases the translation of the mature messenger RNA. Loss of *tat* function results in completely non-infectious virus in which no detectable viral RNA or proteins can be expressed.

The open reading frame of the *rev* protein overlaps with that of the *tat* but the role of *rev* in the regulation of virus production is quite different. *Rev* prevents the transcribed viral RNA from being processed into accessory gene products, thus resulting in increased production of virion structural proteins necessary for assembly of infectious virion. Hence, loss of *rev* function results in the loss of infectious virion assembly capability.

One aspect of the development of agents to inhibit viral infections has focused on prophylactic treatment, *i.e*., a vaccine or molecule which interacts solely with the viral machinery to inhibit virus particle production. The vaccines require the use of inactive virus particles as a source of antigen, which could lead to an allergic response to the contaminating proteins and the potential for incomplete inactivation. Further, viral mutations may require a continuing update of the antigen, as in the case of influenza vaccines.

Other workers have focused on the development of antiviral molecules which inhibit viral replication, particularly in DNA and RNA viruses which utilize their own polymerase for genome replication. The most successful drugs have been acyclovir and ganciclovir, which inhibit herpes simplex virus I and II and cytomegalovirus thymidine kinase. This strategy has been applied to HIV yielding a variety of nucleoside analogs with varying degrees of inhibition of replication. Of these, azidothymidine (AZT) has had success in reducing the production of virus in the bloodstream and increasing T-helper cell population. However, AZT is toxic to bone marrow and other cells and the treatment is dose limited. Further, the use of AZT is often incompatible with other antiinfectious agents (*e.g*. antifungal agents). Thus, treatment of opportunistic infections which result from immunosuppression require that AZT be discontinued until the infection is cleared.

As described, for example, in Journal of Virology 62(10) (1988) 3914-3917, synthetic oligonucleotide therapy has been proposed for the treatment of such viral infections, for example, synthetic deoxyribonucleic acid sequences which are complementary to viral messenger ribonucleic acid (mRNA) have been shown to inhibit virus production in infected cells. This approach employs what has been termed antisense DNA because the synthetic DNA, usually an oligomer 20 to 40 sequences in length (20mer to 40mer), is complementary to (the opposite of) the coding strand of the proviral DNA or the viral mRNA. The use of sense DNA has hitherto not provided effective viral inhibition (see, *e.g.* the above Journal of Virology reference).

The proposed mechanism of action is that the antisense DNA would bind to the viral mRNA resulting in a DNA-RNA duplex. This would result in the degradation (cleavage) of the RNA portion of the duplex by an intracellular enzyme called RNase H. As a result of this cleavage, it would no longer be possible for the mRNA to participate in protein synthesis. The synthetic antisense DNA would then be freed and available to bind to another mRNA strand.

These sequences are being applied to the treatment of disease. The drawbacks have been that oligonucleotides are susceptible to degradative enzymes existing in the circulation, intravenous administration results in rapid clearance from the bloodstream by the kidney, and uptake is insufficient to produce an effective intracellular drug concentration.

Liposomes are particles of subcellular size, comprised of one or more spherical lipid bilayers which surround an internal aqueous space. Drugs may be encapsulated either within the internal aqueous space or in the lipid bilayer, which is usually composed of a phospholipid such as saturated or unsaturated phosphatidylcholine, some type of sterol, and various other, charged or neutral, natural or synthetic lipids. The employment of a liposome delivery system can theoretically overcome the above mentioned problems in the administration of nucleic acid sequences. Liposome encapsulation protects the oligonucleotides from the degradative enzymes, increases the circulation half-life and increases uptake efficiency as a result of phagocytosis of the liposomes.

Phospholipids are amphipathic molecules which are the primary constituents of cell membranes. Typical phospholipid hydrophilic groups include phosphatidylcholine and phosphatidylethanolamine moieties, while typical hydrophobic groups include a variety of saturated and unsaturated fatty acid moieties. Mixture of a phospholipid in water causes spontaneous organization of the phospholipid molecules into a variety of characteristic phases depending on the conditions used. These include bilayer structures in which the hydrophilic groups of the phospholipids interact at the exterior of the bilayer with water, while the hydrophobic groups interact with similar groups on adjacent molecules in the interior of the bilayer. Such bilayer structures can be quite stable and form the principal basis for cell membranes.

Phospholipid bilayer structures can also be formed into closed spherical shell-like structures which are called phospholipid vesicles or liposomes. The membrane bilayers in these structures typically encapsulate an aqueous volume, and form a permeability barrier between the encapsulated volume and the exterior solution. Phospholipids dispersed in aqueous solution spontaneously form bilayers with the hydrocarbon tails directed inward and the polar headgroups outward to interact with water. Simple agitation of the mixture usually produces multilamellar vesicles (MLVs), structures with many bilayers in an onion-like form having diameters 1-10 »m (1000-10,000 nm). Sonication of these structures, or other methods known in the art, leads to formation of unilamellar vesicles (UVs) having an average diameter of about 30-300 nm. However, the range of 50 to 100 nm is considered to be optimal from the standpoint of, e.g., maximal circulation time *in vivo*. The actual equilibrium diameter is largely determined by the nature of the phospholipid used and the extent of incorporation of other lipids such as cholesterol. Standard methods for the formation of liposomes are known in the art, for example, methods for the commercial production of liposomes are described in U.S. Patent No. 4,753,788 to Ronal C. Gamble and Patent No. 4,935,171 to Kevin R. Bracken, the disclosures of which are incorporated herein by reference.

Either as MLVs or UVs, liposomes have proven valuable as vehicles for drug delivery in animals and in humans. Active drugs, including small hydrophilic molecules and polypeptides, can be trapped in the aqueous core of the liposome, while hydrophobic substances can be dissolved in the liposome membrane. The liposome structure can be readily injected and form the basis for both sustained release and drug delivery to specific cell types, or parts of the body. MLVs, primarily because they are relatively large, are usually rapidly taken up by the reticuloendothelial system (the liver and spleen).

UVs, however, tend to remain in the circulatory system for hours and break down after internalization by the target cell. Liposome mediated delivery of antisense DNA and DNA analogs have been used in attempts to inhibit viral proliferation (see, for example, DE-A-3742049).

### Summary of the Invention

While the prior art has demonstrated the principle of using antisense DNA to inhibit virus production, the present invention comprises the delivery of sense DNA in association with a phospholipid delivery vehicle, preferably in liposome encapsulated form, to inhibit virus proliferation in infected cells and to serve as a prophylactic treatment to uninfected cells. Preferably, the sense oligonucleotide is encapsulated in UVs having a diameter of less than 200 nm, most preferably less than 100 nm.

Broadly, the method for the inhibition of viral replication in a cell comprises the intracellular delivery of a DNA oligonucleotide having the same sequence as a DNA coding strand of viral origin. Preferably, the sense oligonucleotide has the same sequence as the viral DNA coding strand for the production of a viral regulatory protein, particularly a *trans*-activating protein, and most preferably the DNA coding strand is a splice site necessary for the production of infectious virions. Significant advantages are obtained in a method in which the regulatory protein is *tat* or *rev*, in which the splice site includes the nucleotide sequence CAG, and in which the DNA oligonucleotide has the sequence 5'-ATTTTCAGAATTGGGTGTCG-3'. The method is applicable to viruses, has shown efficacy in retroviruses, particularly of the retrovividae family and the lentivivinae subfamily, and specifically useful in a method for inhibition of the replication of the human immunodeficiency virus. The invention provides significant advantages in the elimination of toxicity, since the DNA sequence is common only to the virus and has no effect on non-viral organisms, and the DNA sequence of the gene determines the specificity of the therapeutic. The method of the invention can inhibit HIV replication at the step of the conversion from viral RNA to DNA upon initial entry followed by inhibition of integration due to incomplete reverse transcription, or by the prevention of transcription of viral DNA to viral RNA. The sense oligonucleotide can be administered to infected cells *in vitro* or *in vivo* in amounts which are effective to treat the infection.

In addition to liposomes, other phospholipid delivery vehicles may be used to deliver sense oligos, such as those disclosed in the Vestar, Inc. patent publication EP0272091. This patent application, the counterpart of U.S. Serial No. 942,093 filed 15 December 1986, describes the use of a lipid particle which is composed of a single encapsulating phospholipid membrane associated with an amphiphile-associated substrate.

### Detailed Description of the Preferred Embodiment

The invention includes a method for use of sense DNA in the inhibition of viral proliferation in viruses that replicate through the integration of viral DNA, directly from the viral genome or transcribed from viral genomic RNA, into the host cell genome. In particular, the DNA employed is sense to the viral DNA coding strand which codes for the production of a viral regulatory protein.

Such viruses include viruses having accessory protein splice sites in the viral genome, adenovirus, herpes simplex viruses, Epstein Barr viruses, hepatitus virus, and papilloma virus. Examples of such accessory proteins are Ela protein for adenovirus, ICP4 protein for herpes simplex viruses, SM1, Z1 and R1 proteins for Epstein Barr viruses, and protein X for hepatitus virus. Other such viruses include the avian leukemia virus, the murine leukemia virus, the human T-cell leukemia virus, the human cytomegalovirus, and the human immunodeficiency viruses; all of which also include, in the viral DNA, a splice site necessary for the production of infectious virions.

The delivery of sense DNA, *i.e*., an oligonucleotide having the same rather than a complementary sequence to the coding strand, offers several advantages over conventional antiviral treatments in that a sequence can be selected which is specific only to the virus and is therefore non-toxic to the cells. In addition, the selected target sequence can be one which is highly conserved in the viral genome to avoid viral resistance to the treatment as a result of natural viral mutation. While we do not wish to be bound to any particular theory, the synthetic sense DNA appears to be able to inhibit virus production at two stages in the viral cycle. The first consists of the binding of sense DNA to viral RNA directly after the uncoating of the virion. The second is the binding of the sense to the proviral DNA, thus preventing transcription. For the reasons mention above, the use of sense DNA which codes to the sequence for the production of a viral regulatory protein, a *tran*s-activating protein, or a splice site necessary for the production of infectious virions is particularly advantageous in double-stranded DNA and RNA viruses.

Sense DNA which inhibits production of the *tat* protein is advantageous in cells infected with HIV because the elimination of this protein completely inhibits viral replication. Furthermore, *tat* has been shown to enter non-infected cells and inhibit the stimulation of immune cells by itself. Hence, by inhibiting *tat* protein production not only is the production of infectious virions inhibited, but also the secondary inhibition of the immune system by *tat* itself is inhibited.

In the examples which follow, a DNA sequence from the 5' splice acceptor site of the HIV-1 (human immunodeficiency virus) *trans*-activating protein termed *tat* was selected. The phosphodiester antisense sequence has been shown to inhibit HIV proliferation as determined by assaying for reverse transcriptase activity which is a characteristic enzyme activity of the virus. Inhibition of the HIV *tat* protein is highly desirable because upon production, this protein binds to the 5' long terminal repeat sequence of the integrated proviral DNA resulting in approximately 100 fold increase in viral protein production. Furthermore, since it is a regulatory gene, the copies of viral message should be much lower than copies of mRNA for the HIV structural genes (proteins which make up the virion).

The sense and the antisense sequences were encapsulated in small unilamellar liposomes composed of dipalmitoylphosphatidylcholine (DPPC), cholesterol (CHOL) and N-(N-ethylsuccinimidylthio)distearoylphosphatidylethanolamine (NEST-DSPE) in a ratio of 48/25/27 mol/mol. Specifically, a phosphatidylethanolamine (distearoylphosphatidylethanolamine (DSPE)) is attached to an acetylthio group by the addition of succinimidylacetylthioacetate (SATA) to form a phosphatidylethanolaminoacetylthioacetate (PE-ATA) moiety, specifically distearoylphosphatidylethanolaminoacetylthioacetate (DSPE-ATA). Liposomes are then formed (in ways which are within the knowledge of those of ordinary skill in the art) consisting of DPPC/Chol/DSPE-ATA, that is, liposomes consisting of DPPC/Chol/DSPE with an -ATA group extending externally from the surface thereof. After liposome formation, the -ATA moiety is deacetylated by addition of either hydroxylamine or ammonium hydroxide, and the free sulfhydryl of the -ATA is then reacted with N-ethylmaleimide to form the succinimidyl moiety on the DSPE, *e.g*., the NEST-DSPE. The succinimidyl moiety, extending from the surface of the liposome as the only significant extending group (in the absence of extending ligand conjugates such as hormones or other proteins or peptides which extend from the lipid particle, *e.g*., for the purpose of inducing a metabolic response in a target cell upon the binding of the ligand conjugate to its receptor), provides significant advantages in that it enables the cytoplasmic or nuclear cell delivery of intact oligonucleotides, to avoid degradation of the agents by the cell lysosome. Further information regarding this liposome formulation is found in the copending application entitled *Phospholipid Analogue Vesicle* which is filed on even date herewith.

Only the liposomes containing the sense sequence to the *tat* splice acceptor site inhibited virus production. The degree of inhibition has ranged from 50% to 85%. Furthermore, a 50% reduction in the number of infected cells was observed. Hence, the liposomes containing the sense DNA to the 5' splice acceptor site of the HIV *tat* gene were able to reduce viral production and inhibit the spread of the virus to uninfected cells.

### EXAMPLES

### Synthesis of DSPE-ATA

DSPE-ATA is synthesized by a reaction which generates a modified phosphatidylethanolamine having an acetylthioester group on the ethanolamine moiety. For example, a five-fold molar excess of SATA is added to DSPE in a round bottom flask. Typically, 125 mg SATA is added to 75 mg of DSPE. 15 ml of CHCl₃:MeOH (1:1) is then added, followed by 100-135 »l of triethylamine. The flask is then flushed with nitrogen, sealed, and the reaction carried on for two hours at room temperature with stirring. The progress of the reaction is monitored by thin layer chromatography in CHCl₃:MeOH (7:3). The absence of a Ninhydrin positive, and the presence of a PMA positive spot having an Rf of 0.57 indicates the presence of DSPE-ATA. Some of this compound may oxidize to the disulfide (ATA-PE-ATA-PE), which has an Rf of about 0.37. It should be noted that SATA is quite labile and cannot be stored for prolonged periods. The material obtained is then evaporated to dryness and resuspended in 1.0 ml of CHCl₃:MeOH (1:1), and 15 ml of acetonitrile is added to the dry material. This solution is held at -20°C for about 60 minutes to precipitate DSPE-ATA. The precipitate is collected by filtration on a sintered glass filter and washed with acetonitrile. The washed DSPE-ATA is again dissolved by the addition of 1:1 CHCl₃:MeOH and collected in a second filter flask, transferred to a preweighed flask and evaporated. The yield at this point has been between 80 and 84%.

During preparation, all steps should be conducted as quickly as possible to minimize oxidation. However, the removal of oxidized DSPE-ATA is accomplished by preparative thin layer chromatography, such as on a Kieselgel 60 plate obtained from EM Science. Aluminum backed silica gel (no fluorescent indicator) plates are used with CHCl₃:MeOH (7:3) as the mobile phase. DSPE-ATA is located by I₂ staining a strip cut from the end of the plate. The area containing DSPE-ATA is then cut out. The strips of SiO₃-coated aluminum are further cut up and extracted over a 30 minute period in 1:1 CHCl₃:MeOH. The DSPE-ATA is concentrated to dryness, dissolved in 1:1 CHCl₃:MeOH and dispensed into vials. Each vial is flushed with nitrogen and stored at -20°C.

### Liposome Production

A solution containing 15 »mol of the lipids DPPC, DSPE-ATA and CHOL (49/27/25) was prepared in chloroform. The chloroform was evaporated under a stream of nitrogen to yield a dry powder or lipid film. The film was vacuum desiccated overnight to remove residual organic solvent. One film was hydrated in 0.4 ml of phosphate buffered saline (PBS). Second and third films were hydrated in PBS containing either 2.5 mg of synthetic sense DNA (5'-ATTTTCAGAATTGGGTGTCG-3') or 2.5 mg of synthetic antisense DNA (5'-CGACACCCAATTCTGAAAAT-3'). For preparation of liposomes by extrusion, samples were brought through three freeze and thaw cycles using liquid nitrogen and thawing at 65°C. The suspensions were extruded through a 0.4um, 0.2 »m and 0.1 »m polycarbonate filters three times per filter at 65°C and at a maximum of 800 psi to form liposomes having an average diameter of 100 nm. Each sample was extruded through a set of filters separately to avoid contamination and the order of extrusion was PBS, antisense DNA and sense DNA. Argon was bubbled through the lipid suspensions to remove oxygen. The thioacetylated lipid was deacetylated by addition of hydroxylamine. The byproducts were separated by a sephadex G-25 spin column and the samples were incubated for 2.5 hours at room temperature. To attach a succinimide moiety to the liposome surface, N-ethylmaleimide was added to the liposome dispersion in a mol/mol excess of ten to one with respect to the thiolated lipid and incubated overnight at 4°C to yield the N-ethylsuccinimidylthiol phospholipid analogue. The byproducts were removed by a biogel A5M gel filtration column. The lipid fractions were pooled and sterile filtered using a 0.2 polycarbonate nucleopore filter. The preparation yielded liposomes of less than 100 nm in diameter, which contained approximately 140 molecules of each DNA per liposome.

### Tests of Viral Inhibition

The sterile liposome preparations produced as described in the preceding paragraph were added to human peripheral blood mononuclear cells (PBMCs) which had been cultured for three days in phytahemagglutinin (PHA). PBMCs from HIV positive infected patients were cultured with conditioned media to produce virus in the supernatant. This supernatant was collected and used to infect the PHA stimulated uninfected PBMCs. The liposomes were added 3 hours after the PBMCs were treated with the infected supernatant. Virus production was monitored by assaying the cell supernatant for HIV core protein termed *gag* protein or abbreviated p24. Levels of p24 were determined by a standard ELISA assay. The number of infected cells were determined by immunochemically treating fixed infected cultures with antibody to the HIV p17 and gp41 proteins (which also react with gp160 protein) followed by a secondary antibody to which an enzyme was linked. The infected cells were visualized by then incubating with a colored substrate to the enzyme followed by staining the uninfected cells.

The liposomes containing the 5'-ATTTTCAGAATTGGGTGTCG-3' DNA (having the same sequence as the DNA coding strand of viral origin, *i.e*., the sense DNA) reduced the production of p24 into the cell supernatant by 85% with respect to the untreated control whereas liposomes containing the 5'-CGACACCCAATTCTGAAAAT-3' (having the opposite sequence of the DNA coding strand of viral origin, *i.e*., the antisense DNA) showed no inhibition of p24 production. Immunocytochemical staining of the infected cells showed that the liposomes containing the sense sequence reduced the number of strongly positive infected cells by 50% whereas the liposomes containing the antisense sequence showed no reduction in the number of strongly positive cells. It was also observed that the untreated control and liposomes containing the antisense sequence displayed large cell aggregates whereas the liposomes containing the sense sequence showed a greatly reduced number of large cell aggregates (approximately 30% of the untreated control). Neither the liposomal sense or liposomal antisense DNA sequence displayed cell toxicity as assayed by cell viability.

In a second set of experiments, free DNA was compared to liposomal DNA described above using two doses and testing for breakthrough of the virus on day 5 and day 7. Samples containing 50 and 100 »M lipid were tested, which was equivalent to a concentration of 17 and 34 nM DNA. Liposomes containing the sense sequence showed 48% inhibition of p24 production at 50 »M lipid and 50% inhibition at 100 »M lipid on day 5 of infection. On day 7 the 50 »M lipid showed 28% p24 inhibition and 31% inhibition at 100 »M lipid. Liposomes containing the antisense sequence showed only 1.2% inhibition with 50 mM lipid and -9.6% inhibition with 100 »M lipid on day 5. Day 7 showed 8% inhibition with 50 »M and 5% inhibition with 100 »M lipid.

Free 5'-ATTTTCAGAATTGGGTGTCG-3' (sense) yielded -8% inhibition with 17 nM DNA and 2% inhibition with 34 nM. 17% inhibition was observed for 17 nM and 13% inhibition was observed for 34 nM DNA on day 7. Empty liposomes showed no inhibition of p24 production. Furthermore, immunocytochemical staining for infected cells showed a decrease in the number of strongly positive infected cells and the number of giant cells by treatment with liposomes containing the sense DNA sequence compared to that observed for free sense, empty liposomes or liposomes containing the antisense sequence.

Finally, in a third set of experiments, DNA analogs of the above sequences were prepared containing modified 3' and 5' ends to increase resistance to nuclease degradation. These liposomes were prepared by first codissolving the DNA and lipid in chloroform/methanol (1:1) at a ratio of 0.5 mg DNA to 10 »mol lipid. The solvent was evaporated forming a film on the side of the test tube, and the film was vacuum desiccated overnight to remove residual solvent. The films were hydrated in 150 mM NaCl, 10 mM phosphate buffer. The hydrated films were then brought through three freeze and thaw cycles followed by the standard extrusion protocol previously described. Analysis showed a 23% trapping efficiency.

Empty liposomes formed by this method were tested against similar liposomes containing the above-described sense and antisense sequences. Liposomes were tested at 2, 10 and 50 »M lipid and analyzed for inhibition of p24 production on day 5 and day 7. The following results were obtained for the sense liposomes containing the low, medium and high dose: on day 5: -8%, 11% and 42% of untreated control, respectively and on day 7: -6%, 28% and 60% of the untreated control. For the antisense liposomes, the results obtained for the low, medium and high dose on day 5 were 4%, 1% and 8% of the untreated control, respectively, and for day 7 the results were 2%, 11%, and 16% of the untreated control. Blocking the 3' and 5' ends of the oligonucleotides prevented oligonucleotide degradation and virus breakthrough on day 7, and shows the sequence specificity of the the splice site sense strand for the viral inhibition.

Nonspecific inhibition was observed for the empty liposomes as well, yielding 1%, 11% and 15% of the untreated control on day 5 and -60%, 18% and 28% of the untreated control on day 7 for the low, medium and high lipid doses.

Thus, liposomal sense DNA significantly reduces viral antigen production, p24 levels, and the number of infected cells. DNA-free liposomes displayed some non-specific inhibition, which appears to account for the apparent inhibition seen by the liposomal antisense formulation, but the inhibition was significantly less than that shown by the sense liposomes.

## Claims

1. Use of a sense DNA oligonucleotide, having the same sequence as a DNA strand which codes for the production of a viral regulatory protein in a virus having a genome which becomes integrated in the host DNA of a cell, in association with a phospholipid delivery vehicle, in the preparation of a composition for the intracellular delivery of the said sense DNA oligonucleotide to the cell for inhibiting the proliferation therein of the virus.

2. The use of claim 1 in which the DNA coding strand is a splice site necessary for the production of infectious virions.

3. The use of claim 1 or 2 in which the viral regulatory protein for which the DNA coding strand codes is a *trans*-activating element of viral origin.

4. The use of claim 3 in which the regulatory protein is *tat* or *rev*.

5. The use of any one of claims 1 to 4 in which the splice site includes the nucleotide sequence CAG.

6. The use of claim 1 in which the DNA oligonucleotide has the sequence 5'-ATTTTCAGAATTGGGTGTCG-3'.

7. The use of claim 1 in which the virus is selected from the group consisting of adenovirus, herpes simplex virus, hepatitis virus, papilloma virus, leukaemia virus, cytomegalovirus and the human immunodeficiency virus.

8. The use of any one of claims 1 to 7 in which the DNA oligonucleotide is encapsulated in liposomes as the phospholipid delivery vehicle.

9. Use of liposomes for containing a sense DNA oligonucleotide, having the same sequence as a DNA coding strand which codes for the production of a viral regulatory protein in a virus having a genome which becomes integrated in the host DNA of a cell, to be delivered intracellularly to the cell for inhibiting the proliferation therein of the virus.

## Patentansprüche

1. Verwendung eines gleichsinnigen DNS-Oligonucleotids , das dieselbe Sequenz wie ein DNS-Strang aufweist, der die Produktion eines viralen Regulationsproteins in einem Virus codiert, das ein Genom besitzt, welches in die Wirts-DNS einer Zelle integriert wird, zusammen mit einem Applikaitonsvehikel aus Phospholipid, bei der Herstellung einer Zusammensetzung zur intrazellularen Applikation des besagten gleichsinnigen DNS-Oligonucleotids in die Zelle zur Hemmung der Fortpflanzung des Virus in ihr.

2. Verwendung nach Anspruch 1, wobei der codierende DNS-Strang eine zur Herstellung infektiöser Virionen nötige Spleißstelle ist.

3. Verwendung nach Anspruch 1 oder 2, wobei das virale Regulationsprotein, das der codierende DNS-Strang codiert, ein *trans*-aktivierendes Element viralen Ursprungs ist.

4. Verwendung nach Anspruch 3, wobei das Regulationsprotein *tat* oder *rev* ist.

5. Verwendung nach einem der Ansprüche 1 bis 4, wobei die Spleißstelle die Nucleotidsequenz CAG umfaßt.

6. Verwendung nach Anspruch 1, wobei das DNS-Oligonucleotid die Sequenz 5'-ATTTTCAGAATTGGGTGTCG-3' aufweist.

7. Verwendung nach Anspruch 1, wobei das Virus aus der Gruppe, bestehend aus dem Adenovirus, dem Herpes simplex-Virus, dem Hepatitisvirus, dem Papilloma-Virus, dem Leukämievirus, dem Cytomegalovirus und dem menschlichen Immunschwächevirus, gewählt wird.

8. Verwendung nach einem der Ansprüche 1 bis 7, wobei das DNS-Oligonucleotid in Liposome als Applikationsvehikel aus Phospholipid eingekapselt wird.

9. Verwendung von Liposomen als Behälter eines gleichsinnigen DNS-Oligonucleotids, das dieselbe Sequenz wie ein codierender DNS-Strang hat, der die Produktion des viralen Regulationsproteins in einem Virus codiert, das ein Gemom besitzt, das in die Wirts-DNS einer Zelle integriert wird, um der Zelle intrazellulär appliziert zu werden, zur Hemmung der Fortpflanzung des Virus in ihr.

## Revendications

1. Utilisation d'un oligonucléotide d'ADN sens, ayant la même séquence qu'un brin d'ADN qui code pour la production d'une protéine régulatrice de virus dans un virus ayant un génome, qui s'intègre dans l'ADN hôte d'une cellule, en association avec un véhicule de livraison de phospholipides, dans la préparation d'une composition pour la livraison intracellulaire dudit oligonucléotide d'ADN sens à la cellule pour y inhiber la prolifération du virus.

2. Utilisation selon la revendication 1, dans laquelle le brin codant l'ADN est un site d'épissage nécessaire à la production de virions infectieux.

3. Utilisation selon la revendication 1 ou 2, dans laquelle la protéine régulatrice de virus, pour laquelle code le brin codant l'ADN, est un élément de transactivation d'origine virale.

4. Utilisation selon la revendication 3, dans laquelle la protéine régulatrice est tat ou rev.

5. Utilisation selon l'une quelconque des revendications 1 à 4, dans laquelle le site d'épissage comprend la séquence nucléotidique CAG.

6. Utilisation selon la revendication 1, dans laquelle l'oligonucléotide d'ADN possède la séquence 5'-ATTTTCAGAATTGGGTGTCG-3'.

7. Utilisation selon la revendication 1, dans laquelle le virus est choisi parmi l'ensemble comprenant les adénovirus, le virus herpès simplex, le virus de l'hépatite, le virus du papillome, le virus de la leucémie, les cytomégalovirus et le virus de l'immunodéficience humaine.

8. Utilisation selon l'une quelconque des revendications 1 à 7, dans laquelle l'oligonucléotide d'ADN est encapsulé dans des liposomes sous forme d'un véhicule de livraison de phospholipides.

9. Utilisation de liposomes pour qu'il contiennent un oligonucléotide d'ADN sens, ayant la même séquence qu'un brin codant l'ADN, qui code pour la production d'une protéine régulatrice de virus dans un virus ayant un génome qui s'intègre dans l'ADN hôte d'une cellule, pour livraison intracellulaire à la cellule dans le but d'y inhiber la prolifération du virus.
